# EUROPEAN PATENT SPECIFICATION

(11) **EP 2 046 309 B1**
(45) Date of publication and mention of the grant of the patent: **15.12.2010**
(21) Application number: 07804636.4
(22) Date of filing: 24.07.2007
(51) Int. Cl.: A61K 31/00, A61K 31/593, A61P 37/08, A61P 11/06, A61P 17/00

(54) **USE OF VITAMIN D3 AGONIST MC903 IN A MAMMALIAN MODEL FOR ATOPIC DISEASES**
VERWENDUNG EINES VITAMIN-D3-AGONISTEN MC903 BEI EINEM SÄUGERMODELL FÜR ATOPISCHE ERKRANKUNGEN
UTILISATION D'UN AGONISTE DE VITAMIN D3 MC903 DANS UN MODELE MAMMIFERE POUR LEA MALADIES ATOPIQUES

(30) Priority: 24.07.2006 EP 06291201; 24.07.2006 US 832864 P
(43) Date of publication of application: 15.04.2009
(73) Proprietor: Association pour la recherche à l'IGBMC (ARI), 67000 Strasbourg (FR)
(72) Inventor: CHAMBON, Pierre, F-67113 Blaesheim (FR); METZGER, Daniel, F-67200 Strasbourg (FR); LI, Mei, 67400 Illkirch (FR)
(74) Representative: Novagraaf Technologies
(86) International application number: PCT/IB2007/002102
(87) International publication number: WO 2008/012645

(56) References cited:
- LI MEI ET AL: "Topical vitamin D3 and low-calcemic analogs induce thymic stromal lymphopoietin in mouse keratinocytes and trigger an atopic dermatitis." PROCEEDINGS OF THE NATIONAL ACADEMY OF SCIENCES OF THE UNITED STATES OF AMERICA. 1 AUG 2006, vol. 103, no. 31, 1 August 2006 (2006-08-01), pages 11736-11741, XP002410505 ISSN: 0027-8424
- LEHMANN B ET AL: "Vitamin D and skin: New aspects for dermatology" EXPERIMENTAL DERMATOLOGY, SUPPLEMENT 2004 UNITED KINGDOM, vol. 13, no. 4, 2004, pages 11-15, XP009075656 ISSN: 1479-1250
- KATAYAMA I ET AL: "Topical vitamin D3 downregulates IgE-mediated murine biphasic cutaneous reactions." INTERNATIONAL ARCHIVES OF ALLERGY AND IMMUNOLOGY. SEP 1996, vol. 111, no. 1, September 1996 (1996-09), pages 71-76, XP009075657 ISSN: 1018-2438
- KATAYAMA I ET AL: "Topical vitamin D3 (tacalcitol) for steroid-resistant prurigo." THE BRITISH JOURNAL OF DERMATOLOGY. AUG 1996, vol. 135, no. 2, August 1996 (1996-08), pages 237-240, XP009075658 ISSN: 0007-0963
- KATAYAMA I ET AL: "Topical glucocorticoid augments IgE-mediated passive cutaneous anaphylaxis in Balb/C mice and mast cell deficient WBB6F1 v/v mice." CLINICAL AND EXPERIMENTAL ALLERGY : JOURNAL OF THE BRITISH SOCIETY FOR ALLERGY AND CLINICAL IMMUNOLOGY. DEC 1997, vol. 27, no. 12, December 1997 (1997-12), pages 1477-1483, XP009075659 ISSN: 0954-7894

## Description

The present application claims priority to U.S. Provisional Patent Application No.: 60/832,864 filed on July 24, 2006 and European Patent Application N° EP 06291201.9 filed on July 24, 2006.

### Field of the Invention

This invention relates to the field of atopic diseases, in particular to atopic dermatitis (AD).

### Background

Human atopic diseases are major health problems and comprise atopic dermatitis (AD), asthma, and allergic rhinitis (SPERGEL and PALLER, J. Allergy Clin. Immunol., vol. 112 (6 Suppl), p: S118-27, 2003).

Human atopic dermatitis (AD) is a chronic skin inflammatory disease with a strong genetic component that affects children (10-20%) and adults (1-3%) (LEUNG et al., J. Clin. Invest., vol.113, p:651-657, 2004). Atopic dermatitis results notably in skin eczematous-like lesions with xerosis and pruritus, associated with a skin inflammatory infiltrate mainly composed of CD4+ T helper type 2 (Th2) cells, dendritic cells, eosinophils and mast cells, and systemic abnormalities including elevated serum IgE and IgG levels, as well as blood and tissue eosinophilia.

Asthma is a disorder characterized by lung inflammation (with elevated eosinophils, Th2 cells and macrophages, as well as Th2-type cytokines), intermittent reversible airway obstruction, airway hyperreactivity (AHR), excessive mucus production, and elevated serum levels of IgE and Th2-type cytokines. It affects approximately 5% of the population in the Western world and its incidence is increasing dramatically in developed nations (RENAULD, J. Clin. Pathol., vol.54(8), p:577-89, 2001; ELIAS et al., J. Clin. Invest., vol.111 (3), p:291-7, 2003). It is noteworthy that approximately half of AD patients will develop asthma at later stages of their life, often associated with severe AD ( SPERGEL and PALLER, 2003, abovementioned).

Nuclear receptors (NRs) belong to a superfamily of ligand-dependent transcriptional regulators (LAUDET, & GRONEMEYER, The nuclear receptor : factsbook, Academic Press, San Diego, 2002 ; MANGELSDORF et al., Cell, vol.83, p :835-839, 1995). Within this superfamily, Retinoid X Receptors (RXRs) α, β and γ play a key role through heterodimerization with some 15 NR partners, e.g. Retinoic Acid Receptors (RARs) α, β and γ, Vitamin D Receptor (VDR), Peroxisome Proliferator-Activated Receptors (PPARs) and liver X receptors (LXRs).

The inventors have previously reported (LI et al., Proc. Natl. Acad Sci. USA, vol.102, p:14795-14800, 2005) that selective ablation of RXRα and RXRβ in adult mouse epidermal keratinocytes (RXRαβ^{ep-/-} mice) triggers a skin and systemic phenotype similar to human atopic dermatitis (AD). In fact, these mice exhibit the major features of the human AD syndrome that includes (i) skin eczematous-like lesions with xerosis and pruritus, associated with a skin inflammatory infiltrate mainly composed of CD4+ T helper type 2 (Th2) cells, dendritic cells, eosinophils and mast cells, and (ii) systemic abnormalities including elevated serum IgE and IgG levels, and blood and tissue eosinophilia. The inventors also established that expression of the cytokine thymic stromal lymphopoietin (TSLP), known to be produced in epidermal keratinocytes of AD patients (SOUMELIS et al., Nat. Immunol., vol.3, p :673-680, 2002), is rapidly induced in keratinocytes of RXRαβ^{ep-/-} mice. Furthermore, the inventors showed that K14-TSLP transgenic mice overexpressing TSLP in keratinocytes exhibit an AD-like phenotype similar to that of RXRαβ^{ep-/-} mice (L1 *et al.,* 2005, above-mentioned), demonstrating that TSLP can act as an initiating cytokine at the top of a chain of immunological events that lead to an AD-like phenotype. In addition the inventors have recently shown that ablation of the TSLP gene in mice prevents the generation of an AD-like phenotype (unpublished data), demonstrating that TSLP expression is both sufficient and necessary for generating an AD in the mouse. In keeping with these results others have shown that in human asthmatic airways TSLP expression is increased and correlated with both the expression of Th2-attracting chemokines and with disease severity. Moreover, lung-specific expression of a TSLP transgene induced asthma-like syndrome, whereas, in contrast, mice lacking the TSLP receptor (TSLPR) failed to develop asthma in response to inhaled antigens (LIU, J. Exp. Med., vol.203, p :269-273, 2006 : ZHOU et al.., Nat. Immunol., vol.6, p:1047-1053, 2005 ; YOO et al., J. Exp. Med., vol.202, p:541-549, 2005; AL-SHAMI et al. J. Exp. Med., vol.202, p:829-839. 2005; YING et al., J. Immunol., vol.174(12), p:8183-8190. 2005). The presence of TSLP in epidermis and lung epithelium is therefore necessary and sufficient to trigger in the mouse an AD-like phenotype and an asthma-like syndrome, respectively.

However, the molecular mechanism underlying the induction of TSLP expression remains unknown, thus precluding the development of novel mouse models in which the generation of human-like atopic diseases would allow screening for drugs which could be used for human atopic disease treatment through interference with the physiological mechanism that controls TSLP expression.

### Summary of the Invention

This invention provides a method for generating a human atopic dermatitis-like phenotype in a non human mammal comprising the step (i) of administrating to said mammal 26,27-cyclo-22-ene-1α,24S-dihydroxyvitamin D3.

In a preferred embodiment, said step (i) further comprises administrating to said mammal at least one compound selected in the group comprising natural and synthetic Retinoic Acid Receptor (RAR) agonists, preferably a RARγ-selective agonist.

In a still preferred embodiment, said method further comprises the step (ii) of assessing the generation of atopic dermatitis-like phenotype in said mammal.

In another preferred embodiment, said method further comprises the step (iii) of administrating to said mammal a compound that can be useful for treating and/or preventing atopic disease.

In still another preferred embodiment, said method further comprises the step (iv) of analyzing the human atopic dermatitis-like phenotype of the mammal with or without the administration of the compound that has been identified to be possibly useful for treating and/or preventing a human atopic dermatitis disease.

In still another preferred embodiment, said method further comprises the step (v) of selecting the compounds that revert and/or prevent the human atopic dermatitis-like phenotype.

The present also discloses a method for treating and/or preventing an atopic disease in a patient comprising the step (i) of administrating to said patient an effective amount of at least one vitamin D3 antagonist.

In a preferred embodiment, said method for treating and/or preventing atopic disease further comprises the step (ii) of administrating to said patient an effective amount of at least one RAR antagonist.

### Detailed Description

As RXR/NR heterodimers in which an agonistic ligand is not bound to the NR partner can act as transcriptional repressors (PERISSI and ROSENFELD, Nat. Rev. Mol. Cell. Biol., vol.6. p:542-554, 2005), the inventors have examined whether TSLP expression could be modified by NR agonists.

Among the tested NR agonists, the active derivatives of vitamin D3 and vitamin A have been considered.

Vitamin D3 is a secosteroid, which is involved in the control of a wide variety of biological processes in higher animals. These processes include maintenance of calcium homeostasis, immunomodulation and selected cell differentiation. Vitamin D3, itself, is biologically inactive. However, metabolism of vitamin D3 to the biologically active compound 1,25-Dihydroxyvitamin D3 [1α,25-(OH)₂D₃] is responsible for the wide array of biological responses which are observed as part of the vitamin D endocrine system.

Vitamin A (retinol) is an essential component of the diet. Both clinical and experimental approaches have shown that vitamin A derivatives (retinoids) are necessary for normal growth, vision, and maintenance of numerous tissues, reproduction and overall survival (BLOMHOFF, Nutr. Rev., vol.52(1 Pt 2), p:S13-23, 1994; SPORN et al., The retinoids. Biology, Chemistry and Medecine., New York: Raven 1994). Vitamin A is also known to play an important role in immune responses (STEPHENSEN, Annu. Rev. Nutr., vol21, p:167-92, 2001). The active derivative of Vitamin A is retinoic acid (RA). Experimentally one can manipulate retinoid levels in vivo by providing vitamin A deficient diets or by administering RA or its synthetic agonistic analogs.

Recently it has been suggested that vitamin D and vitamin A could possibly be used to treat AD patients (WORM, Curr. Opin. Investig. Drugs, vol.3. p :1596-1603. 2002 : LEHMANN et al., Exp. Dermatol., vol. 13 (Suppl 4), p:11-15, 2004 : ZASLOFF, J. Invest. Dermato/., vol.125, p :xvi-xvii, 2005 ; ZASLOFF, Nat. Med., vol.12, p :388-390,2006).

On the contrary, the inventors have now established that administration of active derivatives of these vitamins generates an AD-like phenotype in the mouse, and therefore may exacerbate AD in patients, by promoting expression of TSLP in epidermis.

Consequently, a first object of the present invention concerns a method for generating a human atopic dermatitis-like phenotype in a non human mammal comprising the step (i) of administrating to said mammal 26,27-cyclo-22-ene-1α,24S-dihydroxyvitamin D3 (MC903).

Said non human mammal is preferably a rodent, and most preferably a mouse.

As used herein, the term "atopic disease" refers to a disease selected in the group comprising atopic dermatitis (AD), asthma and allergic rhinitis.

As used herein, the term "agonistic analog" refers to a compound that mimics at least part of the functions of an agonistic ligand, preferably an endogenous agonistic ligand, by binding to its receptor. As used in the present invention, the term "agonistic analog" refers more particularly to a compound that binds the Vitamin D receptor (VDR), and induces at least some biological activities of the endogenous ligand 1α25(OH)₂D₃.

As used herein, the term "antagonist analog" refers to an inhibitor (full or partial) of either an agonistic ligand, preferably an endogenous agonistic ligand, thereby blocking at least a part of the physiological activity of the agonist. As used in the present invention, the term "antagonist analog" refers more particularly to a compound that binds the Vitamin D receptor (VDR) and blocks biological activities of the endogenous ligand 1α25(OH)₂D₃.

In the present invention said atopic disease is atopic dermatitis.

Preferably, said compound is administrated to the skin of said mammal, preferably to the ear skin.

In addition, said method further comprises the step of (ii) assessing the generation of a human AD-like phenotype. Such assessing step can be realized by external skin aspect observation, skin histological examination (i.e., presence of eosinophils, mast cells and dendritic cells), analyzing TSLP and Th2 type cytokine expression in skin (e.g., by northern blots and quantitative RT-PCR (Q-PCR) for RNA transcripts, or by western blot and immunohistochemistry for proteins), and analyzing TSLP and IgE serum levels (e.g., by ELISA), and blood eosinophilia.

The term "physiologically active vitamin D3 (1α,25 (OH)₂ D₃)" as used therein, refers to the 1,25-Dihydroxyvitamin D3.

Vitamin D3 agonistic analogs are known from one of skill in the art and include, as non-limiting examples, 1α,18,25-(OH)₃D₃, 23-(m-(Dimethylhydroxymethyl)-22-yne-24,25,26,27(teranor)-1α-OH)₂D₃, 1α,25-Dihydroxy-trans-Isotachysterol(1,25-trans-Iso-T), (1S,3R,6S)-7,19-Retro-1,25-(OH)₂D₃, (1S,3R,6R)-7,19-Retro-1,25-(OH)₂D₃, 22-(p-(Hydroxyphenyl)-23,24,25,26,27-pentanor-D₃, 22-(m-(Hydroxyphenyl)-23,24,25,26,27-pentanor-D₃ described in PCT application WO 95/17197, 26, 27-cyclo-22-ene-1α,24S-dihydroxyvitamin D3 (MC903), 1(S),3(R)-dihydroxy-20(R)-(5'-ethyl-5'-hydroxy-hepta-1'(E),3'(E)-dien-1'-yl)-9,10-secopregna-5(Z),7(E),10(19)-triene (EB1089), and 1α,25-(OH),-20-epi-22-oxa-24,26,27-trishomovitamin D (KH1060) described in CARLBERG et al. (J. Steroid. Biochem. Mol. Biol., vol.51, p :137-142, 1994), and 1R,25-dihydroxy-21-(3-hydroxy-3-methylbutyl)vitaminD₃ described in CARLBERG et al. (J. Cell Biochem., vol.88, p:274-281, 2003).

Vitamin D3 is also implicated in calcium homeostasis and its administration to a mammal may result in hypercalcemia.

Advantageously, said vitamin D3 agonistic analog is a low-calcemic vitamin D3 agonistic analog. Such low-calcemic vitamin D3 agonistic analogs are known from one of skill in the art and include, as non-limiting examples, a low-calcemic vitamin D3 synthetic agonistic analog selected in the group comprising 26, 27-cyclo-22-ene-1α,24S-dihydroxyvitamin D3 (MC903), 1(S),3(R)-dihydroxy-20(R)-(5'-ethyl-5'-hydroxy-hepta-1'(E),3'(E)-dien-1'-yl)-9,10-secopregna-5(Z),7(E),10(19)-triene (EB1089), 1α,25-(OH),-20-epi-22-oxa-24,26,27-trishomovitamin D (KH1060), and 1R,25-dihydroxy-21-(3-hydroxy-3-methylbutyl)vitaminD₃.

The inventors have demonstrated that TSLP, which acts as an initiating cytokine at the top of a chain of immunological events leading to an AD-like phenotype, is induced by the administration of 1α,25 (OH)₂ D₃ or of MC903 to ear skin.

Therefore, said vitamin D3 agonistic analog has to induce TSLP expression and corresponds, to 26, 27-cyclo-22-ene-1α,24S-dihydroxyvitamin D3 (MC903).

One of skill in the art can simply determine the effective amount of MC903 thereof to be administrated in order to induce an human AD-like phenotype in view of its general knowledge and of the protocols described in the examples.

As an example, such effective amount for vitamin D3 is comprised in the range of 0.025 nmole to 4 nmoles per cm² of skin, preferably in the range of 0.1 nmole to 0.5 nmole per cm² of skin.

As an example, such an effective amount for the MC903 vitamin D3 agonistic analog is comprised in the range of 0.025 nmole to 5 nmoles per cm² of skin, preferably in the range of 0.5 nmole to 2.5 nmole.

The inventors have also demonstrated that topical administration of a RAR agonist, or preferably a RARγ-selective agonist, boosts the TSLP increase induced by 1α,25 (OH)₂ D₃ administration.

In another embodiment, the step (i) further comprises administrating to said mammal of at least one compound selected in the group comprising natural and synthetic Retinoic Acid Receptor (RAR) agonists.

Natural and synthetic RAR agonists are well known from one of skill in the art and include, as examples, retinoic acid (RA) as a natural RAR agonist, and synthetic RAR agonists such as the RARγ-selective racemic mixture of R- and S-3-fluoro-4-[2-hydroxy-2-(5,5,8,8-tetramethyl-5,6,7,8,-tetrahydro-naphthalen-2-yl)-acetulamino]-benzoic acid (BMS961; BOURGUET & GERMAIN *et al., Trends Pharmacol. Sci.,* vol.21(10), p: 381-8, 2000), the pan-RAR agonist (R)-3-Fluoro-4-[2-hydroxy-2-(5,6,7,8-tetrahydro-5,5,8,8-tetramethylnaphthalen-2-yl)-2-acetylamino]benzoic Acid (BMS270394; KLAHOLZ et al., Proc. Natl. Acad. Sci. U S A, vol.97, p: 6322-6327, 2000 ; KLAHOLZ et al., J. Mol. Biol., vol.302, p : 155-170, 2000), and the pan-RAR agonist (E)-4-[2-(5,6,7,8-Tetrahydro-5,5,8,8-tetramethylnaphthalen-2-yl)prop-1-en-1-yl]benzoic Acid (TTNPB; THACHER, VASUDEVAN et al.., Curr. Pharm. Des., vol.6(1), p: 25-58, 2000).

One of skill in the art can simply determine the effective amount of natural or synthetic RAR agonist to be administrated with MC903 in order to induce an AD-like phenotype, in view of his general knowledge and of the protocols described in the examples.

As an example, such effective amount for BMS961 is comprised in the range of 0.025 nmole to 5 nmoles per cm² of skin, preferably in the range of 0.5 nmole to 2.5 nmoles.

In another preferred embodiment, said administration step corresponds to a daily administration and is maintained for a sufficient period to induce an atopic disease, preferably an atopic dermatitis.

As an example said sufficient period to induce an atopic disease, preferably an atopic dermatitis, corresponds to a daily administration for a period comprised between 1 and 30 days, preferably three days and one month, and more preferably between 10 and 20 days, and can be resumed at any time thereafter.

In still another preferred embodiment, the method of the invention further comprises the step (iii) of administrating to said mammal a compound that could be useful for treating and/or preventing an atopic disease. Thus, the method of the invention enables to identify compounds that could be useful for treating and/or preventing an atopic disease, preferably atopic dermatitis (AD).

As used herein, "treating and/or preventing" an atopic disease means that symptoms of atopic disease are prevented, reduced or inhibited after the administering of said compound.

The administrating steps of (i) 26,27-cyclo-22-ene-1α, 24S-dihydroxyvitamin D3, and of (iii) a compound that could be useful for treating and/or preventing an atopic disease can be realized simultaneously or successively.

The term "compound that could be useful for treating and/or preventing an atopic disease", as used herein, refers to any compound such as a polypeptide, an oligonucleotide, a polysaccharide, a vitamin D3 antagonist or a RAR antagonist.

Advantageously, said compound is a vitamin D3 antagonist or an RAR antagonist, and more preferably a vitamin D3 antagonist.

More advantageously, said compound is a combination of a vitamin D3 antagonist and of RAR antagonist.

Preferably, said at least one vitamin D3 antagonist and at least one RAR antagonist are administrated simultaneously or successively.

Vitamin D3 antagonists are well known from one of skill in the art and include, as examples, the vitamin D3 antagonistic analogs selected in the group comprising 14-Epi-1,25-(OH)₂D₃, 14-Epi-1,25-(OH)₂-Pre-D₃, 1,25-(OH)₂-7,8-cis-D₃, 1,25-(OH)₂-5,6-trans-7,8-cis-D₃ described in PCT application WO 95/17197, the vitamin D3 antagonists described in PCT application WO 02/15894, and butyl-(5Z,7E,22E)-(1S,3R,24R)-1,3,24-trihydroxy-26,27-cyclo-9,10-secocholesta-5,7,10(19),22-tetraene-25-carboxylate (ZK159222), (23S)-25-dehydro-1α(OH)D3-26,23-lactone (TEI 9647), and ZK168281 as described in CARLBERG (2003, abovementioned).

Preferably, said vitamin D3 antagonistic analog selectively antagonizes the effect of the physiologically active vitamin D3 on the immune system.

RAR antagonists are also well known from one of skill in the art and include, as examples, Pan-RAR antagonists selected in the group comprising 4-(6-Methoxyethoxymethoxy-7-adamantyl naphthalen-2-yl)benzoic Acid (CD2665; SZONDY et al., Mol. Pharmacol., vol.51(6), p: 972-82, 1997), 4-[2-(5,6-dihydro-5,5-dimethyl-8-p-tolylnaphthalen-2-yl)ethynyl]benzoic acid (AGN193109; KLEIN et al., J. Biol. Chem., vol.271(37), p: 22692-6, 1996) and (E)-4-[2-[5,6-Dihydro-5,5-dimethyl-8-(2-phenylethynyl)naphthalene-2-yl]ethen-1-yl]benzoic Acid (BMS493; GERMAIN et al., Nature, vol.415(6868), p: 187-92, 2002).
In a preferred embodiment, said method further comprises the step (iv) of analyzing the human atopic disease-like phenotype of the mammal with or without the administration of the compound that has been identified to be possibly useful for treating and/or preventing a human atopic disease.
In a still preferred embodiment said method further comprises the step (v) of selecting the compounds that revert and/or prevent the human atopic disease-like phenotype.

Preferably, said RAR antagonist is a RARγ-selective antagonist.

The selected compounds may be then further tested for their toxicity and/or their ability to prevent or treat atopic disease in other animal models.
The present invention has also for object a method for aggravating asthma by generating a human atopic disease-like phenotype by applying the method of the present invention for generating a human atopic dermatitis previously in a non human mammal model of experimental asthma.
In preferred embodiment the method for aggravating asthma by generating a human atopic disease-like phenotype further comprises an assessing step realized by lung histopathological examination, analysis of bronchoalveolar lavage (BAL) fluid, lung TSLP and Th2-type expression, and by physiological test of lung function.
The present invention has also for object the use of non human asthma mammal model obtained by said a method for aggravating asthma in a screening method.
In preferred embodiment the screening method is for screening any compounds useful for preventing and/or treating severe asthma.

Preferably the compound selected in the group comprising the physiologically active vitamin D3 and its agonistic analogs and eventually compounds that can be useful for treating and/or preventing asthma are administrated to lungs. Methods for administrating a compound to the lungs are well known from one of skill in the art and include aerosol inhalation.

Advantageously, said method further comprises the step of (ii) assessing the generation of asthma. Such assessing step can be realized by lung histopathological examination (e.g., identification of lung inflammation), analysis of bronchoalveolar lavage (BAL) fluid, lung TSLP and Th2-type cytokine expression, and by physiological tests of lung function (e.g. measurement of airway hyperresponsiveness as described in RANGASAMY et al. (J. Exp. Med., vol.202(1), p: 47-59 2005), and in Animal Models of Airway Sensitization (Unit 15.18 in Current Protocols in immunology, John Wiley & Sons, Inc., 1999)).

The present also discloses a method for treating and/or preventing an atopic disease in a patient comprising the step of administrating to said patient an effective amount of at least one vitamin D3 antagonist.

The term "patient" as used herein refers to a mammal, preferably to a human.

Preferably, said atopic disease is atopic dermatitis (AD) or asthma, and more preferably atopic dermatitis (AD).

More preferably, said method further comprises the step of administrating to said patient at least one RAR antagonist.

The at least one vitamin D3 antagonist and at least one RAR antagonist can be administrated simultaneously or successively.

The at least one vitamin D3 antagonist with or without at least one RAR antagonist can be formulated into pharmaceutical compositions (also called "medicaments") comprising a pharmaceutically acceptable carrier. Formulation of pharmaceutical compositions of the invention are within the skill in the art, for example as described in Remington's Pharmaceutical Science, 17th ed., Mack Publishing Company, Easton, Pa. (1985), the entire disclosure of which is herein incorporated by reference.

According to the present, an "effective amount" of at least one vitamin D3 antagonist and possibly of at least one RAR antagonist is one, which is sufficient to achieve a desired biological effect, in this case the prevention or reversion of an atopic disease. It is understood that the effective dosage will be dependent upon the age, sex, health, and weight of the recipient, kind of concurrent treatment, if any, frequency of treatment, and the affinity of said antagonist for its receptor.

The present also discloses a use of a composition comprising at least one vitamin D3 antagonist analog for the manufacture of a medicament for treating and/or preventing an atopic disease in a patient.

Preferably, said atopic disease is atopic dermatitis (AD) or asthma, and more preferably atopic dermatitis (AD).

More preferably, the composition further comprises at least one RAR antagonist.

Preferably, the medicament is administred to the skin or to the lungs of the patient, and more preferably to the skin.

The invention will now be illustrated by the following non-limiting examples.

### EXAMPLES

### 1) Topical application of 1α,25-(OH)2D3 (the physiologically active vitamin D3) or of its low-calcemic analog MC903 induces TSLP expression in epidermal keratinocytes

As RXR/NR heterodimers in which an agonistic ligand is not bound to the NR partner can act as transcriptional repressors (PERISSI and ROSENFELD, 2005, abovementioned), we examined whether TSLP expression could be induced by a variety of agonistic ligands of NRs known to heterodimerise with RXRs.

Four nmoles of ligands dissolved in ethanol were topically applied to whole ears of 6-8-week-old female CD1 wildtype (WT) mice for 4 consecutive days (D1 to D4), and TSLP RNA levels were determined on day 5.

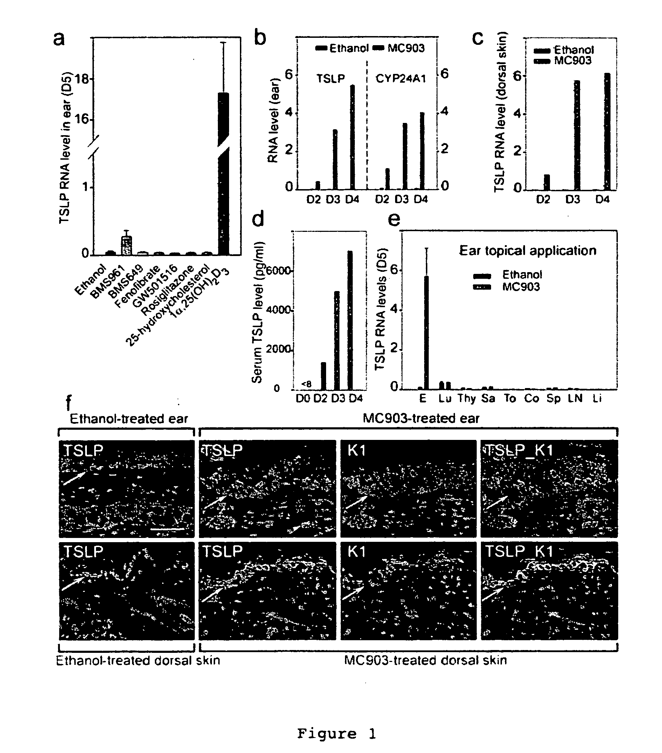

Figure 1a shows the TSLP RNA level (determined by Q-PCR) in ear epidermal keratinocytes at day 5 (D5) after skin topical application of ethanol, BMS961, BMS649, fenofibrate, GW501516, Rosiglitazone and 1α,25(OH)₂D₃.

The results show that topical application of selective agonists for RXRs (BMS649, also known as SR11237; LEHMANN et al., Science, vol.258, p:1944-1946, 1992), PPARα (Fenofibrate) (INOUE et al., Biochem. Biophys. Res. Commun., vol.290, 131-139, 2002), PPARβ (GW501516, OLIVER et al., Proc. Natl. Acad. Sci. USA, vol.98, p:5306-5311, 2001), PPARγ (Rosiglitazone; LEHMANN et al., J. Biol. Chem., vol.270, p:3406-3410, 1997) and LXRs (25-hydroxycholesterol; FOWLER et al., J. invest. Dermatol., vol.120, p: 246-255, 2003) had no effect on TSLP expression (determined by Q-PCR; see Fig. 1a). In marked contrast, application of 1α,25-(OH)2D3 led to a dramatic increase (>300-fold) in TSLP transcripts at D5, whereas they were modestly, but significantly increased (5-fold) upon application of a RARγ-selective agonist (BMS961 ; CHAPELLIER et al., Embo J., vol.21, p :3402-3413, 2002 ; see Fig. 1a).

As, at this dose, 1α,25-(OH)₂D₃ application resulted in hypercalcemia and death of the mice, we applied 1α,25-(OH)₂D₃ every second day at a dose of 0.25 nmole or its analog MC903 (calcipotriol, Dovonex ; CARLBERG, 2003, abovementioned), that exhibits a low calcemic activity and is used for psoriasis treatment (KRAGBALLE and IVERSEN, Dermatol. Clin., vol.11, p :137-141, 1993), every day. 1α,25-(OH)₂D₃, or MC903 and ethanol (vehicle) were applied to WT mouse right and left ears, respectively. Two shaved areas (1cm² each) of dorsal skin were also treated with MC903 or ethanol.

Figure 1b shows the TSLP and CYP24A1 RNA levels (determined by Q-PCR) in ethanol-treated left ear and MC903-treated right ear at day 2, 3 and 4 (D2, D3 and D4) after skin topical application.

Figure 1c shows the TSLP RNA levels (determined by Q-PCR) in ethanol-treated and MC903-treated dorsal skin (c) at D2, D3 and D4. Figure 1d shows the serum TSLP levels (pg/ml) at D0, D2, D3 and D4 (these data are representative of three independent experiments).

The results show that one day after the first application (D2), TSLP RNA levels were increased in right ears, and further increased on D3 and D4, whereas no increase occurred in left ears (see Fig. 1b, left panel). Transcripts of *CYP24A1,* a 1α,25-(OH)2D3-inducible gene (JONES et al., Physiol. Rev., vol.78, p :1193-1231, 1998), were increased upon MC903 application, as expected (see Fig. 1b, right panel). TSLP RNA was also increased in MC903-treated dorsal skin (see Fig.1c), and serum TSLP levels were increased at D2-D4, while undetectable, at D0 (before treatment, see Fig. 1d). Increasing doses of MC903 (0.4, 1 or 4 nmoles per ear) led to a dose-dependent increase of TSLP transcripts, which was similarly observed with other low-calcemic analogs of 1α,25-(OH)2D3, including EB 1089 and KH1060 (CARLBERG, 2003, abovementioned : CARLBERG *et al.,* 1994, abovementioned ; and our data not shown).

To examine whether MC903-induced expression of TSLP was skin-restricted, various other organs were analyzed at D5.

Figure 1e shows the TSLP RNA levels (determined by Q-PCR) at D5 of mice topically treated by ethanol or MC903 on ears : in ear (E), lung (Lu), thymus (Thy), salivary gland (Sa), tongue (To), colon (Co), spleen (Sp), lymph node (LN) and liver (Li).

The results show that no increase in TSLP transcripts was observed in these organs, with the exception of ears (see Fig. 1e).

Figure 1f shows the expression of TSLP and keratin 1 (K1) by immunochemistry at D4 in sections of ear (upper panels) and dorsal skin (lower panels) of mice topically treated with ethanol or MC903, as indicated. The white arrowhead points to autofluorescent erythrocytes, and white arrows point to the dermal/epidermal junction. (Scale bar, 50 µm).

Immunohistochemistry (IHC) shown in Fig.1f did not reveal TSLP expression in epidermis and dermis of ethanol-treated ear or dorsal skin, whereas it was readily detected at D4 upon MC903 treatment (see Fig. 1f; ear skin: upper panel; dorsal skin: lower panel). Double IHC for TSLP and keratin 1 (K1, used as a suprabasal keratinocyte marker), showed that TSLP was mainly located in these keratinocytes in both MC903-treated ear and dorsal skin, while it could also be detected at a lower level in basal keratinocytes (expressing keratin 14) (see Fig. 1f, and data not shown).

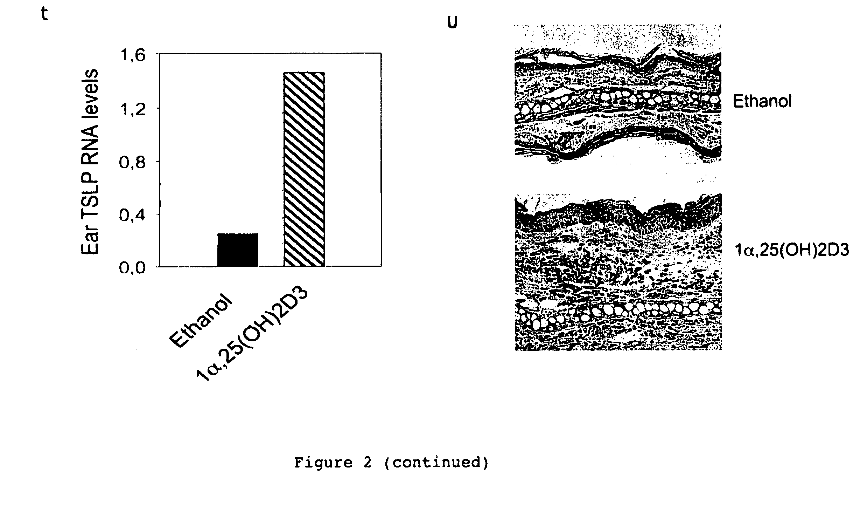

Figure 2t shows TSLP RNA levels (determined by Q-PCR) in ear epidermal keratinocytes at day 18 (D18) after skin topical application every second day of ethanol and 1α,25(OH)₂D₃, at a dose of 0.25 nmole per ear.

The results show that on day 18, TSLP RNA levels were increased in ears (see Figure 2t). No hypercalcemia or overall health impairment and weight loss was observed.

### 2) Topical application of MC903 or of 1α,25(OH)₂D₃ triggers an AD-like syndrome

As TSLP expression appears to be critically involved in the initiation of AD-like dermatitis in the mouse (LI *et al.,* 2005, abovementioned. YOO *et al.,* 2005, abovementioned), we investigated whether a MC903 and 1α,25(OH)₂D₃ long-term treatment could induce an AD-like phenotype.

MC903 (4 nmoles) or 1 α,25(OH)D₃ (0,25 nmole) was applied daily or every second day respectively, for 16 days, to ears of 6-8-week-old female CD1 wildtype (WT) mice. No hypercalcemia or overall health impairment and weight loss was observed. Ethanol application did not cause any change in ear appearance, whereas reddening and swelling, that worsened with time, were observed from D5 on MC903-treated ears or 1α,25(OH)₂D₃ -treated ears (not shown).

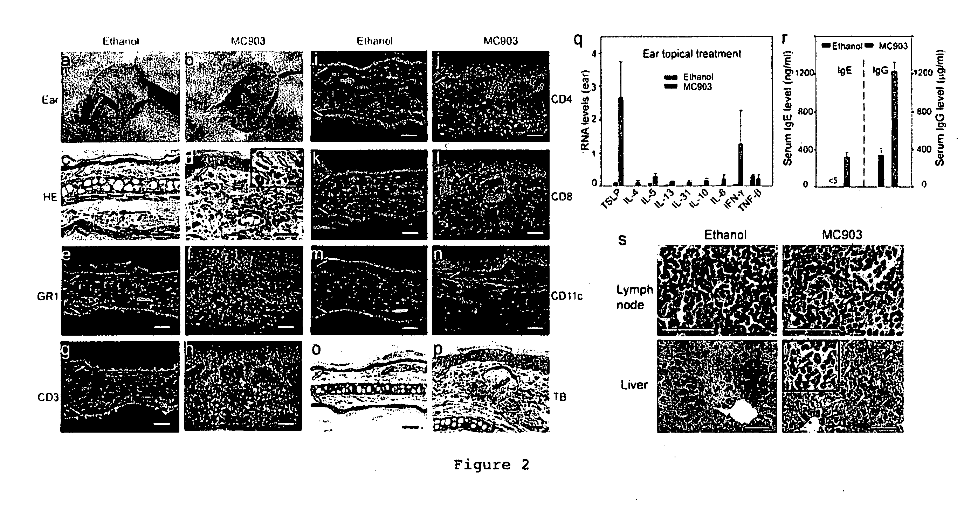

Figure 2 shows the effect of ear topical treatment with MC903, 1α,25(OH)₂D₃ or ethanol. White arrows point to the dermal/epidermal junction.

Figures 2a and b show the ear appearance at day 17 after ethanol and MC903 treatment, respectively.

Figures 2c and d show hematoxylin and eosin-stained (HE) ear sections of ethanol- and MC903-treated mice at day 17, respectively. Eosinophil-selective cytoplasmic red staining is pointed by arrows in the inset of (d).

Figures 2 u show hematoxylin and eosin-stained (HE) ear sections of ethanol- and 1α,25(OH)₂D₃ treated mice every second day, at a dose of 0,25 nmole, at day 18 (D18).

Figures 2 e to n show the result of IHC performed on ear sections from ethanol- or MC903-treated mice at D17, with antibodies against GR1 (e and f), CD3 (g and h), CD4 (i and j), CD8 (k and 1) and CD11c (m and n). Yellow color corresponds to staining of antibodies, whereas blue corresponds to DAPI staining of nuclei.

Figures 2 o and p show the Toluidin blue (TB)-staining of ear sections. Red arrows point to one of the mast cells with intense blue color in the dermis.

Figure 2 q shows cytokine RNA levels (determined by Q-PCR) in ethanol- and MC903-treated ears at D17. The results show the results of ELISA experiments assessing the serum IgE and IgG levels in ethanol- and MC903-treated mice at D17. Figure 2 s show the hematoxylin and eosin-stained sections of ear draining lymph node and liver of ethanol- and MC903-treated mice at D17. Yellow arrows point to three of many eosinophils (red cytoplasmic staining) in sections of lymph node and liver of MC903-treated mice. (Scale bars, 50 µm).

The results shows that at D17, these ears were red, scaly, swollen and crusted (see Fig. 2, compare a and b), while frequent ear scratching (not shown) suggested a pruritus. Histological analysis revealed epidermal hyperplasia and a heavy dermal cell infiltrate, in which numerous eosinophils were easily identified upon hematoxylin/eosin staining (see Fig.2d and inset, and Fig 2u). Their identity was confirmed with eosinophil-selective Luna's staining (not shown). In contrast, no eosinophils were found in ethanol-treated ears (see Fig. 2c and Fig 2u)). 1HC with an anti-GRI antibody (recognizing granulocytes and monocytes) revealed a large number of positive cells in dermis, of which eosinophils but not neutrophils were a major component (see Fig. 2f, and data not shown). Numerous T lymphocytes (CD3+) were observed in MC903-treated dermis (see Fig. 2h), whereas only few resident T lymphocytes could be detected in ethanol-treated ears (see Fig. 2g). Most of the infiltrated T cells were CD4+ helper T cells (see Fig. 2j), and only a few CD8+ cytotoxic T cells were found (see Fig. 2l). A large increase in CD11c+ dermal dendritic cells was also observed in MC903-treated ears (see Fig. 2 m and n), whereas mast cells were 4-fold increased in the dermis (see Fig. 2 o and p, and data not shown).

In WT mice treated every other day with a dose of 0.25 nmole 1α,25-(OH)2D3 per ear, TSLP expression was significantly induced at D18 (See Fig. 2t), and an inflammatory infiltrate comprising CD4+ T lymphocytes, dendritic cells, eosinophils and mast cells could also be observed (Fig.2u, and data not shown).

Taken together, these data indicated that the inflammatory cell infiltrate observed in skin of MC903- and 1α,25-(OH)2D3-treated ears had the characteristics of an AD-like skin inflammation (LI *et al.,* 2005, abovementioned). This was fully supported by Q-PCR analysis of cytokine RNA expressed in MC903-treated ears. At D16, TSLP transcripts were markedly increased (see Fig. 2q), and Th2-type cytokine transcripts (IL-4, IL-5, IL-13, IL-31, IL10 and IL-6) (LI *et al.,* 2005, abovementioned) were all significantly increased (see Fig. 2q). Expression of the Th1-type cytokine IFN-γ was also enhanced, whereas that of TNF-β, another Th1-type cytokine, was unchanged. Importantly, this cytokine expression profile, which is essentially that of a Th2-type inflammation, was similar to those observed in skins of RXRαβ^{ep-/-} and K14-TSLP transgenic mice (LI *et al.,* 2005, abovementioned), indicating that it was most probably due to enhanced TSLP production in keratinocytes. Systemic abnormalities, including elevated serum IgE and IgG levels, associated with blood and tissue eosinophilia, have been observed in RXRαβ^{ep-/-} and K14-TSLP mice, exhibiting similarities to those observed in AD patients (LI *et al.,* 2005, abovementioned). Serum IgE and IgG levels were increased in mice to which MC903 was topically applied for 16 days on ears (see Fig. 2r). Moreover, at D16, MC903-treated mice exhibited an increased number of eosinophils in ear-draining lymph nodes, liver and spleen (see Fig. 2s, and data not shown). Differential blood cell counts also revealed a marked increase in eosinophils in MC903-treated mice (693±220 cells/µl, versus 204±134 cells/µl in ethanol-treated mice). Thus, MC903 topical application leads to a skin and systemic phenotype mimicking that of human AD.

### 3) Both keratinocytic VDR and RXR are required for induction of TSLP expression and generation of an AD-like skin inflammation upon MC903 treatment

To investigate whether the MC903-induced TSLP expression and appearance of an AD-like skin inflammation were mediated through VDR, MC903 was topically applied on ears of "floxed" VDR control (CT) mice (VDR^{L2/L2} mice, in which both VDR alleles bear LoxP sites) and of their VDR^{ep-/-} littermates [K14-Cre^{(tg/0)}/VDR^{L2/L2} mice in which the VDR alleles are selectively ablated in keratinocytes and which were obtained by crossing K14-Cre^{(tg/0)} transgenic mice (LI et al., Development, vol.128, p :675-688, 2001) with floxed VDR^{L2/L2} mice (our unpublished data)].

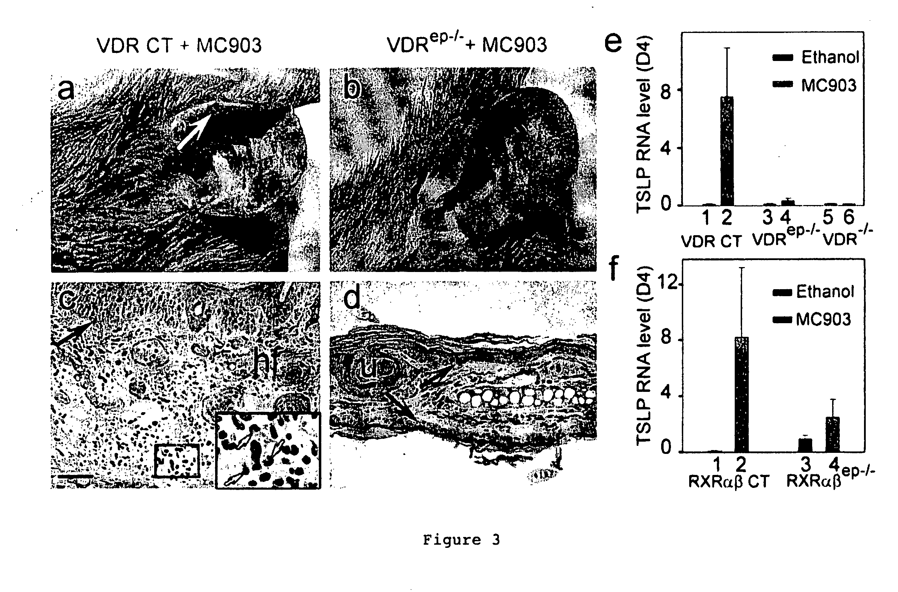

The Figures 3 a and b show the appearance of MC903-treated ears of VDR control (CT) (a) and VDR^{ep-/-} mice (b) at D17. White arrows in (a) points to lesioned skin.

The Figures 3 c and d show Hematoxylin and eosin-stained ear sections. Yellow arrows in inset of (c) point to three of many eosinophils (red cytoplasmic staining) in MC903-treated CT skin. Black arrows point to the dermal/epidermal junction. hf, hair follicle; u, utriculi (resulting from hair follicle degeneration in VDR^{ep-/-} mice). (Scale bar, 50 µm).

Figure 3 e shows TSLP RNA levels (determined by Q-PCR) at D4 in ethanol- and MC903-treated ears of VDR CT (lane 1 and 2), VDR^{ep-/-} (lane 3 and 4) and VDR-/- (lane 5 and 6) mice.

The results show that at D17 of MC903 treatment, an AD-like inflammation was obvious on ears of VDR CT mice, whereas VDR^{ep-/-} ears did not show any sign of inflammation (see Fig. 3a and b). Accordingly, a massive dermal infiltrate of inflammatory cells, including eosinophils, CD4+ T helper cells, dendritic cells and mast cells, was detected in ear sections of MC903-treated VDR CT (see Fig. 3c, and data not shown), but not in those of VDR^{ep-/-} mice (see Fig. 3d). Similarly, no AD-like skin inflammation was developed upon topical MC903 treatment of VDR^{-/-} (germ line knockout) mice (YOSHIZAWA et al., Nat. Genet., vol.16, p :391-396, 1997; data not shown). TSLP expression (determined by Q-PCR) was strongly induced in MC903-treated skin of VDR CT mice (see Fig. 3e, lanes 1 and 2), but not at all in MC903-treated skin of VDR^{-/-} mice (lanes 5 and 6), whereas it was weakly increased in MC903-treated skin of VDR^{ep-/-} mutants (lanes 3 and 4). This latter increase may reflect a faint response to MC903 in non-keratinocytic cells. In any event, our data clearly demonstrated that induction of TSLP expression in keratinocytes upon MC903 application is a VDR-dependent cell-autonomous event that leads to the generation of an AD-like phenotype. In this respect, it is important to note that it has been recently reported that VDR-null mutant mice fail to develop symptoms of experimental asthma (WITTLE et al., J. Immunol., vol. 173, p: 3432-3436, 2004).

To examine whether the effect of MC903 was transduced through RXR/VDR heterodimers, ears of RXRαβ^{ep-/-} mice (tamoxifen-treated K14-Cre-ERT2^{(tg/0)}RXRα^{L2/L2}/RXRβ^{L2/L2} ; LI *et al.,* 2005, abovementioned), as well as their control littermates (RXRαβ CT), were topically-treated with MC903.

Figure 3 f shows TSLP RNA levels (determined by Q-PCR) at D4 in ethanol- and MC903-treated ears of RXRαβ CT (lane 1 and 2) and RXRαβ^{ep-/-} (lane 3 and 4) mice.

As expected (LI *et al.,* 2005, abovementioned), selective RXRαβ-ablation in keratinocytes of adult mice led to increased TSLP expression (lanes 1 and 3). However, the induction of TSLP by MC903 was severely reduced in RXRαβ^{ep-/-} skin (lanes 2 and 4), indicating an essential function of keratinocytic RXRs in TSLP induction by VDR agonists, most probably reflecting the involvement of RXRNDR heterodimers.

### 4) VDR and RARγ agonistic ligands synergize to induce skin TSLP expression

Figure 1a shows that although much less potent than that of the active vitamin D3 [1α,25-(OH)2D3], application of a RARγ-selective agonist (BMS961) on mouse ear skin led to a significant increase in TSLP transcripts. A topical application of RA resulted in a similar induction (data not shown).

To examine whether VDR and RARγ agonists could synergize in upregulating TSLP expression, WT mouse ears were topically-treated for 3 days with either ethanol, BMS961 (4 nmoles), a limiting dose of 1α,25-(OH)2D3 (0.4 nmole), or a combination of the two ligands. Ear TSLP transcripts and serum TSLP were determined at D4.

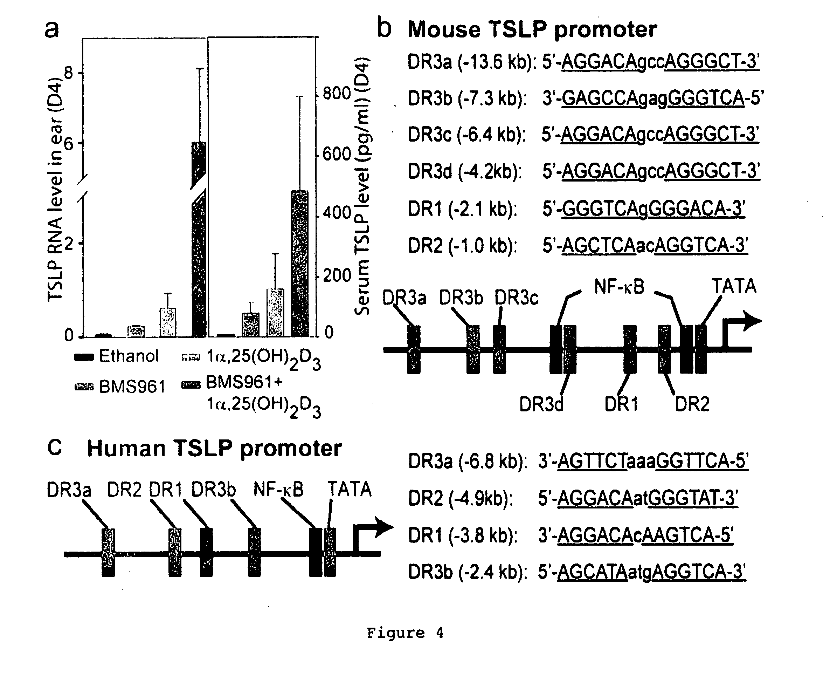

Figure 4 a shows TSLP RNA levels (determined by Q-PCR) in the ears (left panel) and serum TSLP levels (right panel) measured at D4 in mice ear-treated with either Ethanol, BMS961 (4 nmoles), 1α,25(OH)2D3 (0.4 nmoles) or BMS961 (4 nmoles) +1α,25(OH)2D3 (0.4 nmoles).

The results show a clear synergism between the effects of BMS961 and 1α,25-(OH)2D3 (see Fig. 4a), indicating a synergistic involvement of RARγ - and VDR-mediated events in transcriptional activation of TSLP expression. However, on its own, the induction of TSLP expression upon topical treatment with either BMS961 or RA, it too low to trigger an overt AD-like phenotype (data not shown).

Importantly, the fact that agonists of VDR and RARγ could induce TSLP expression either on their own or synergistically, indicates that the corresponding RXR heterodimers bind to distinct cognate response elements.

In this respect, it is noteworthy that both mouse and human TSLP promoter regions contain putative Response Elements (RE) (see Fig. 4b and c respectively) that may bind RXR/VDR heterodimers (VDRE: DR3) or RXR/RAR heterodimers (RARE: DR2 and DR1) (LEID et al., Trends Biochem. Sci., vol.17, p :427-433, 1992).

Based on these evidence, the inventors propose a model accounting for the modulation of TSLP promoter activity by RXRα(β)VDR and RXRα(β)/RARγ heterodimers.

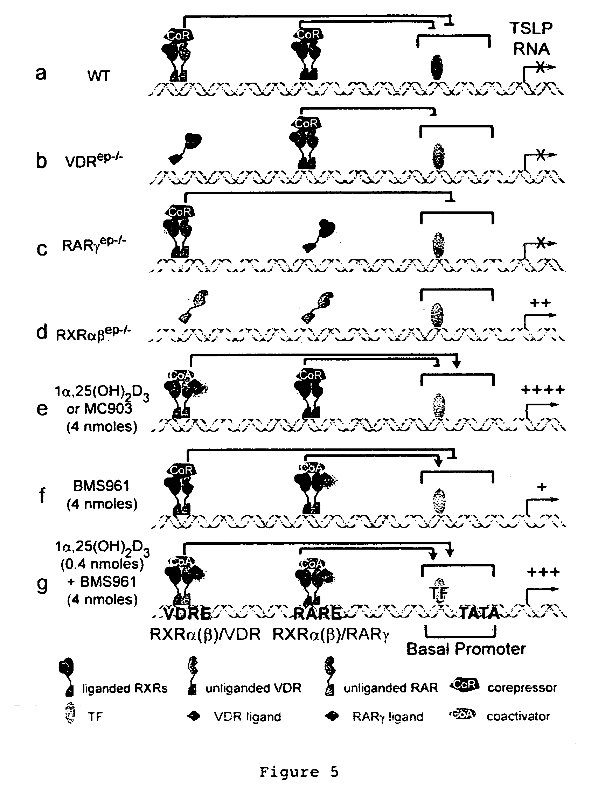

Figure 5 illustrates the schematic model of RXRα(β)VDR- and RXRα(β)/RAR-mediated regulation of TSLP expression in mouse keratinocytes. The promoter region of mouse and human TLSP genes includes a TATA box element and proximal elements (e.g. NF-κB binding sites) (the basal promoter), as well as putative VDREs and RAREs (see Fig. 4b and c).

As under homeostatic conditions in vivo, there is no RA (Calléja et al., Genes & Dev. 20, p1525-1538, 2006) and very little, if any, active vitamin D3 in epidermal keratinocytes, the TSLP promoter basal activity is silenced by unliganded RXRα(β)/VDR and RXRα(β)/RARγ heterodimers associated with corepressors (PERISSI & ROSENFELD, Nat. Rev. Mol. Cell. Biol., vol.6, p :542-554, 2005; see Fig. 5a). This repression can be efficiently exerted by either RXRα(β)/VDR or RXRα(β)/RARγ heterodimers, as it cannot be relieved by ablation of either VDR or RARγ (see Fig. 3e, and data not shown; Fig. 5b and c). On the other hand, RXRα and β ablation (see Fig. 5d), which releases both heterodimers from their binding sites, abolishes this repression and allows basal promoter-bound transcription factors to stimulate TSLP transcription to a basal activity (see Fig. 3f, lane 3) that is sufficient to trigger the generation of an AD-like phenotype (LI *et al.,* 2005, abovementioned).

Topical application of either active vitamin D3 or a low-calcemic analog (MC903) (see Fig. 5e) generates RXR/VDR-coactivator complexes whose transcriptional activity (see Fig. 3f, lane 2) is efficient enough to not only relieve the repression exerted by RXR/RARγ-corepressor complexes, but also to further enhance the basal promoter activity. Interestingly, the RXR/RARγ-coactivator complexes formed upon application of BMS961 are much less efficient (see Fig. 5f), as they generate lower TSLP transcript levels (see Fig. 1a) than those resulting from the basal promoter activity, as observed in keratinocytes ablated for RXRα and β (see Fig. 3f, lane 3). However, upon co-treatment with BMS961 and a limiting dose of 1α,25-(OH)₂D3 (see Fig. 5g), liganded RXR/RARγ and RXR/VDR heterodimers can efficiently synergize to enhance the activity of TSLP basal promoter (see Fig. 4a).

### 5) VDR and RAR antagonists down-regulate Vitamin D3-induced TSLP expression

The rapid and regulable induction of TSLP in mouse keratinocytes upon topical treatment with active Vitamin D3 or low-calcemic vitamin D3 analogs (e.g. MC903) provides a highly convenient AD preclinical model for exploring whether VDR and or RAR antagonists could be used to down-regulate the expression of TSLP.

Vitamin D3 antagonists ZK 168281 (2.5 nmoles) or TEI 9647 (2.5 nmoles) with Ethanol and with or without 1α,25(OH)₂D₃ (0.25 nmole) were daily topically-applied to ears of 6-8-week-old female CD1 (WT) mice. TSLP RNA levels in the ears were measured at day 4 (D4).

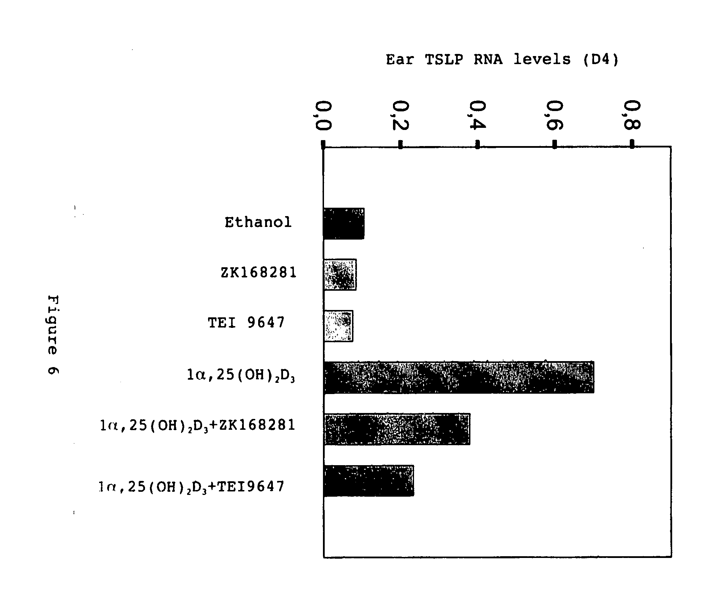

The results show that both of these Vitamin D3 antagonists down-regulate the 1α,25(OH)₂D₃ -induced TSLP expression (see. Figure 6).

The RAR antagonist BMS493 (2.5 nmoles) with ethanol and with or without MC903 (1.25 nmoles) was daily topically-applied to ears of 6-8-week-old female CD1 (WT) mice.TSLP RNA levels in the ears were measured at D3.

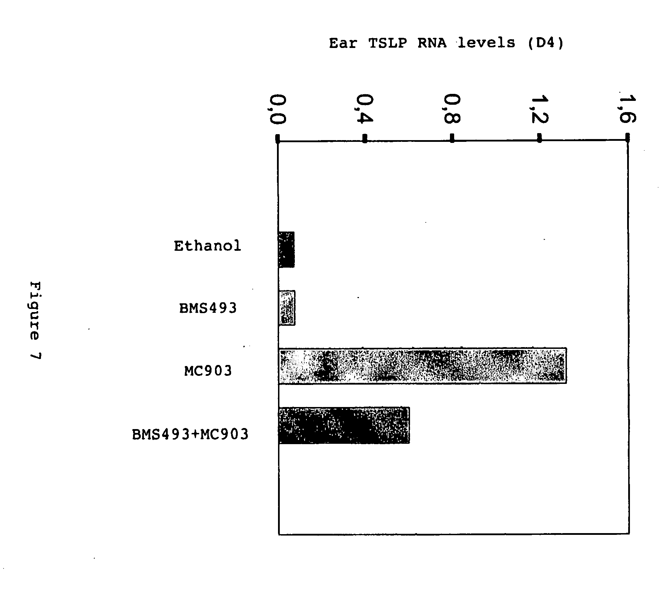

The results show that BMS493 down-regulates MC903-induced TSLP expression (see figure 7), indicating that an RXR/RARγ bound to a RAR antagonist can repress this induction of TSLP expression.

### 6) The asthmatic phenotype of an ovalbumin-induced mouse model is enhanced by agonistic vitamin D3 analogs.

As AD is often the initial step of the "atopic march" that leads to asthma and allergic rhinitis in >30% of AD patients, we investigated whether AD induced by topical application of MC903 on mouse ears could affect the "atopic march", by enhancing the asthmatic phenotype.

An experimental ovalbumin (OVA)-induced asthma mouse model was used. Mice were intraperitoneally injected with 50 µg OVA adsorbed on 2 mg aluminium hydroxide (Alum) on days 0 and 7, and were then challenged on days 18, 19, 20 and 21 by intranasal instillations of 10 µg OVA. As controls, mice were intraperitoneally injected with Alum, and receive intranasal instillations of saline. These mice were topically treated with either ethanol (as a control) or MC903 (1.125 nmole) on each ear every other day from day 0 to day 21 to generate an ear AD.

Assessment of respiratory function, collection of bronchoalveolar lavage (BAL) and lung were performed on day 22.

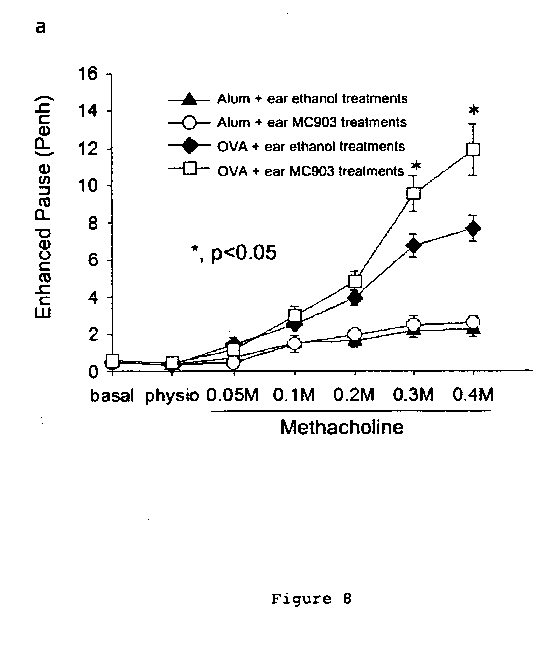

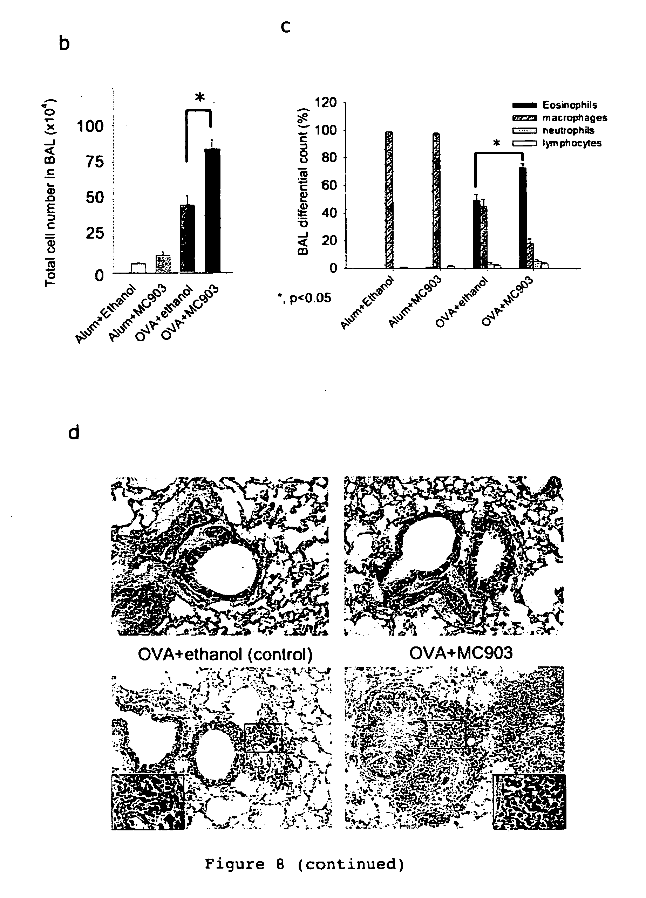

The results show that topical application of MC903 on mouse ear led to both AD (not presented) and to an enhancement of the asthmatic phenotype, including increased airway hyperresponsiveness (AHR) (Figure 8a), higher BAL cell numbers (rich in eosinophils) (Fig.8b and c), increased hypertrophy in airway epithelium, as well as higher perivascular and peribronchial lung inflammatory infiltrations, rich in eosinophils (Figure 8d). Therefore, the present model that associates in the mouse an ear AD with an ovalbumin-induced asthma provides an interesting model to screen for compounds which will be useful for the prevention and treatment of severe asthma.

Figure 8a shows airway hyperresponsiveness (AHR), measured by whole-body plethysmography, of mice treated as indicated and exposed to the indicated concentrations of aerosolized methacholine (0.05M, 0.1M, 0.2M, 0.3M, 0.4M). Aerosolized saline was used as control.

Figure 8b shows the total cell number and figure 8c the percentage of eosinophils, macrophages, neutrophils and lymphocytes at day 22, in BAL of mice treated as indicated.

Figure 8d shows hematoxylin and eosin-stained (HE) lung sections from mice treated as indicated, at day 22. MC903 treatment on ears led to an enhanced airway inflammation, characterized by an increased hypertrophy of the airway epithelium, as well as higher perivascular and peribronchial inflammatory infiltrations in the lung, particularly rich in eosinophils (pointed by arrows in the inset), in OVA sensitized and challenged mice.

A variety of modifications to the embodiments described above will be apparent to those skilled in the art from the disclosure provided herein.

## Claims

1. A method for generating a human atopic dermatitis-like phenotype in a non human mammal, comprising the step (i) of administrating to said mammal 26, 27-cyclo-22-ene-1α24S-dihydroxyvitamin D3 (MC903).

2. The method according to claim 1, further comprising the step of (ii) assessing the generation of atopic dermatitis-like phenotype in said mammal.

3. The method according to claim 2, wherein said assessing step is realized by external skin aspect observation, skin histological examination, analyzing TSLP and Th2 type cytokine expression in skin, analyzing TSLP and IgE serum levels, and analyzing blood eosinophilia.

4. The method according to any of claim 2 to 3, wherein said compound is administrated to the skin

5. The method according to any of claims 1 to 4, wherein said mammal is a mouse.

6. The method according to any of claims 1 to 5, wherein said administration step is a daily administration and said administration step is maintained for a period comprised between 1 and 30 days, and can be resumed at any time thereafter.

7. The method according to any of claims 1 to 6, wherein said method is a method for identifying any compound that can be useful for treating and/or preventing an atopic disease, and wherein said method further comprises the step (iii) of administrating at least one of such compound to said mammal.

8. The method according to claim 7, wherein said method further comprises the step (iv) of analyzing the human atopic dermatitis-like phenotype of the mammal with or without the administration of the compound that has been identified to be possibly useful for treating and/or preventing a human atopic dermatitis.

9. The method according to any of claims 7 to 8, wherein said method further comprises the step (v).of selecting the compounds that revert and/or prevent the human atopic dermatitis-like phenotype.

10. A method for aggravating asthma by generating a human atopic dermatitis-like phenotype according to any of claim 1 to 6 in a non human mammal model of experimental asthma.

11. A method according to claim 10 further comprising an assessing step realized by lung histopathological examination, analysis of bronchoalveolar lavage (BAL) fluid, lung TSLP and Th2-type expression, and by physiological test of lung function.

12. Use of non human asthma mammal model obtained by the method of claim 11 in a screening method.

13. Use according to claim 12, wherein the screening method is for screening any compounds useful for preventing and/or treating severe asthma.

## Patentansprüche

1. Verfahren zur Erzeugung eines Phänotyps des Typs menschliche atopische Dermatitis bei einem nicht menschlichen Säugetier, das den Schritt (i) der Verabreichung von 26,27-Cyclo-22-Ene-1α,24S-Dihydroxyvitamin D3 (MC903) an besagtes Säugetier enthält.

2. Verfahren gemäß Anspruch 1, das des Weiteren den Schritt der Einstufung (ii) der Erzeugung des Phänotyps des Typs atopische Dermatitis in besagtem Säugetier enthält.

3. Verfahren gemäß Anspruch 2, wobei besagter Einstufungsschritt mittels der äußeren Beobachtung des Aussehens der Haut, der histologischen Untersuchung der Haut, der Analyse des TSLP- und des Cytokin-Ausdrucks des Typs Th2 in der Haut, der Analyse des Serumspiegels von TLSP und IgE im Blut und der Analyse der Eosinophilie im Blut durchgeführt wird.

4. Verfahren gemäß einem beliebigen der Ansprüche 2 bis 3, bei dem besagte Verbindung der Haut verabreicht wird.

5. Verfahren gemäß einem beliebigen der Ansprüche 1 bis 4, wobei besagtes Säugetier eine Maus ist.

6. Verfahren gemäß einem beliebigen der Ansprüche 1 bis 5, bei dem der Verabreichungsschritt eine tägliche Verabreichung ist und besagter Verabreichungsschritt während einer Zeitspanne von 1-30 Tagen aufrecht erhalten wird und zu jedem beliebigen späteren Zeitpunkt wieder aufgenommen werden kann.

7. Verfahren gemäß einem beliebigen der Ansprüche 1 bis 6, wobei das Verfahren ein Verfahren zur Identifizierung einer beliebigen Verbindung ist, die zur Behandlung und/oder Vorbeugung einer atopischen Krankheit nützlich sein kann und besagte Methode außerdem einen Schritt (iii) der Verabreichung an besagtes Säugetier von mindestens einer derartigen Verbindung enthält.

8. Verfahren gemäß Anspruch 7, wobei besagtes Verfahren des Weiteren einen Schritt (iv) der Analyse des Phänotyps des Typs menschliche atopische Dermatitis des Säugetiers mit oder ohne Verabreichung der Verbindung enthält, die als möglicherweise nützlich für die Behandlung und/oder Vorbeugung menschlicher atopischer Dermatitis identifiziert worden ist.

9. Verfahren gemäß einem beliebigen der Ansprüche 7 bis 8, wobei besagte Methode des Weiteren einen Schritt (v) der Selektion von Verbindungen enthält, die den Phänotyp des Typs menschliche atopische Dermatitis beseitigen und/oder vorbeugen.

10. Verfahren zur Verschlechterung von Asthma durch Erzeugung eines Phänotyps des Typs menschliche atopische Dermatitis gemäß einem beliebigen der Ansprüche 1 bis 6 in einem nicht menschlichen Säugetiermodell für experimentelles Asthma.

11. Verfahren gemäß Anspruch 10, das des Weiteren einen Einstufungsschritt enthält, der mittels der histopathologischen Untersuchung der Lunge, der Analyse der bronchioalveolären Lavage (BAL)-Flüssigkeit, des TSLP und Th2-Typs Ausdrucks der Lunge, und eines physiologischen Test der Lungenfunktion durchgeführt wird.

12. Verwendung eines nicht menschlichen Asthma-Säugetiermodells, das mittels der Methode gemäß Anspruch 11 in einem Screening-Verfahren erhalten wird.

13. Verfahren gemäß Anspruch 12, wobei die Screening-Methode zum Screening von beliebigen zur Vorbeugung und/oder Behandlung von schwerem Asthma nützlichen Verbindungen dient.

## Revendications

1. Procédé pour générer un phénotype du type dermatite atopique humaine chez un mammifère non humain comprenant l'étape (i) d'administration audit mammifère du 26,27-cyclo-22-ène-1α,24S-dihydrovitamine D3 (MC903).

2. Procédé selon la revendication 1 comprenant en outre l'étape (ii) d'évaluation de la génération du phénotype du type dermatite atopique dans ledit mammifère.

3. Procédé selon la revendication 2, dans lequel ladite étape d'évaluation est réalisée par observation externe de l'aspect de la peau, examen histologique de la peau, analyse de l'expression TSLP et de la cytokine type Th2 dans la peau, analyse des niveaux sériques de TLSP et de IgE, et analyse de l'éosinophilie du sang.

4. Procédé selon l'une quelconque des revendications 2 à 3, dans lequel ledit composé est administré à la peau.

5. Procédé selon l'une quelconque des revendications 1 à 4, dans lequel le mammifère est une souris.

6. Procédé selon l'une quelconque des revendications 1 à 5, dans lequel l'étape d'administration est une administration journalière et ladite étape d'administration est maintenu pendant une période comprise entre 1 et 30 jours, et peut être reprise à n'importe quel temps par la suite.

7. Procédé selon l'une quelconque des revendications 1 à 6, dans lequel ladite méthode est une méthode pour identifier n'importe quel composé qui peut être utile pour le traitement et/ou la prévention d'une maladie atopique, et dans lequel ledit procédé comprend en outre une étape (iii) d'administration d'au moins un de ces composés audit mammifère.

8. Procédé selon la revendication 7, dans lequel ledit procédé comprend en outre l'étape (iv) d'analyse du phénotype du type dermatite atopique humaine du mammifère avec ou sans l'administration du composé qui a été identifié pour être possiblement utile pour le traitement et/ou la prévention de la dermatite atopique humaine.

9. Procédé selon l'une quelconque des revendications 7 à 8, dans lequel ledit procédé comprend en outre l'étape (v) de sélection des composés qui renversent et/ou préviennent le phénotype du type dermatite atopique humaine.

10. Procédé d'aggravation de l'asthme par génération d'un phénotype du type dermatite atopique humaine selon l'une quelconque des revendication 1 à 6 chez un modèle mammifère non humain d'asthme expérimental.

11. Procédé selon la revendication 10 comprenant en outre une étape d'évaluation réalisée par examen histopathologique du poumon, analyse du fluide de lavage broncho-alvéolaire (BAL), l'expression pulmonaire de TSLP et du type Th2, et test physiologique de la fonction pulmonaire.

12. Utilisation d'un mammifère modèle non humain d'asthme obtenu par le procédé selon la revendication 11 dans un procédé de criblage.

13. Utilisation selon la revendication 12, dans lequel la méthode de criblage est pour le criblage de n'importe quel composé utile pour la prévention et/ou le traitement d'asthme sévère.
